(19) Europäisches Patentamt
European Patent Office
Office européen des brevets

(11) **EP 2 676 684 B1**

(12) **EUROPÄISCHE PATENTSCHRIFT**

(45) Veröffentlichungstag und Bekanntmachung des Hinweises auf die Patenterteilung:
**07.01.2015   Patentblatt 2015/02**

(51) Int Cl.:
*A61L 27/00* *(2006.01)*   *C22C 19/00* *(2006.01)*

(21) Anmeldenummer: **13167779.1**

(22) Anmeldetag: **15.05.2013**

(54) **Kobaltlegierung für medizinische Implantate und Stent aus der Legierung**

Cobalt alloy for medical implants and stent comprising the alloy

Alliage de cobalt pour des implants médicaux et stent constitué de l'alliage

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.06.2012   US 201261660824 P**

(43) Veröffentlichungstag der Anmeldung:
**25.12.2013   Patentblatt 2013/52**

(73) Patentinhaber: **Biotronik AG**
**8180 Bülach (CH)**

(72) Erfinder: **Gerold, Bodo**
**97753 Karlstadt (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(56) Entgegenhaltungen:
**EP-A2- 1 604 692     EP-B1- 1 604 691**
**GB-A- 2 230 536     US-A- 2 977 224**
**US-A- 3 713 175**

• **BARBARA HUIBREGTSE ET AL: "New DES Platforms. Does the metal alloy matter?", CARDIAC INTERVENTIONS TODAY, BMC, Nr. July/August, 1. Juli 2011 (2011-07-01) , Seiten 35-39, XP009173263,**

## Beschreibung

Die Erfindung betrifft Co-Basislegierungen, deren dominierender Verformungsmechanismus Zwillingsbildung (TWIP-Effekt) ist. Die Erfindung betrifft ferner einen Stent, der ganz oder in Teilen aus einer Kobaltlegierung besteht.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die vorrangig zur Abdichtung des Aneuryrismas dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Geweveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit permanenten Implantatwerkstoffen, insbesondere Basislegierungen des Kobalts.

Stents müssen die Fähigkeit besitzen große plastische Dehnungen zu ertragen und ihre Größe bzw. Durchmesser zu behalten, wenn sie aufgeweitet sind. Grundsätzlich gilt, der ideale Stent sollte:

- ein geringes Profil besitzen; Dies umschließt die Tauglichkeit auf einen Ballonkatheter gecrimped zu werden.
- ein gutes Aufweitverhalten zeigen; Wenn der Stent in die Läsion eingeführt und der Ballon aufgeblasen wird, soll sich der Stent gleichmäßig aufweiten, um sich der Gefäßwand anzupassen.
- eine ausreichende Radialfestigkeit und einen vernachlässigbaren Recoil besitzen; Wenn der Stent einmal gesetzt ist, soll er den Rückstellkräften der atherosklerotischen Gefäßwand widerstehen und nicht kollabieren.
- eine ausreichende Flexibilität besitzen; So kann der Stent auch durch Gefäße und Stenosen mit geringen Durchmesser bzw. enge Radien befördert werden.
- eine angemessene Röntgensichtbarkeit bzw. MRI-Kompatibilität besitzen; So kann der Mediziner die Implantation und Lage des Stents in vivo beurteilen.
- geringe Thrombogenität besitzen; Das Material sollte biokompatibel sein und insbesondere die Anlagerung und Verklumpung von Blutplättchen vermeiden.
- die Möglichkeit zur Wirkstofffreisetzung haben; Dies dient insbesondere der Restinosevermeidung.

Diese Anforderungen sprechen insbesondere die mechanischen Eigenschaften des Materials an, aus dem der Stent gefertigt ist. Die klassische Materialien 316L, MP53N und L-605, die für den Bau ballonexpandierbarer Stents verwendet werden, haben mechanische Nachteile, die die Freiheit bei der Stent-Design-Entwicklung und im Einsatz einengen:

(i) zu geringe (Zug-)Festigkeit und plastische Dehnung
In der Folge sind der Kollapsdruck bzw. die Radialfestigkeit geringer, so dass dickere Stent-Streben notwendig sind, was zu einem dickeren Crimpingprofil und größeren Lumenverlust bei der Implantation führt, die Einheilung (Endothelialisierung) in die Gefäßwand verzögert und die Freiheiten bei der geometrischen Stent-Design-Entwicklung eingeschränkt. Durch dickere Streben wird der Stent zudem steifer und sein Profil (Durchmesser) im gecrimpten Zustand wird größer, was die Kurvengängigkeit innerhalb des Gefäßsystems herabsetzt.

(ii) zu hohe Dehngrenze Rp0.2
Es resultiert eine starke elastische Rückfederung, was die Crimpbarkeit verschlechtert, zu einem dickeren Crimpprofil führt, die Stenthaltekraft auf dem Ballon verringert und einen höheren Recoil (Lumenverlust nach Aufweitung) bedingt.

Weiterhin muss die Biokompatibilität des Materials sichergestellt sein. Dabei wird immer wieder Nickel als Verursacher von Allergien oder lokalen sowie systemischen Unverträglichkeiten angeführt. Es besteht dem entsprechend ein Bedarf an nickelfreien Werkstoffen oder zumindest Werkstoffen mit geringem Nickelgehalt für den medizinischen Einsatz.

DE 197 04 530 A1 beschreibt eine nickelfreie, austenitische Kobalt-Basislegierung zur Vermeidung von Allergien bei verschiedenen Gebrauchsgegenständen, darunter auch Implantate, mit hoher Korrosionsbeständigkeit und guter Umformbarkeit. Nickel als Stabilisator für den austenitischen Zustand wird hier durch Zugabe von Titan und/oder Niob (zusammen 4 - 6 Gew.%) ersetzt. Die Legierung enthält zudem Cr (10 - 18 Gew.%), Fe (5 - 20 Gew.%) sowie Mo und W (zusammen 4 - 8 Gew.%, wobei der Gehalt an W halb so groß ist, wie der von Mo). Weiterhin kann die Legierung Cu (0 - 2 Gew.%), Mn (0 - 3 Gew.%), A1 (0 - 3 Gew.%), Si (0 - 1 Gew.%), C (0 - 0.1 Gew.%) und N (0 - 0.1 Gew.%) enthalten. Nachteile dieser Legierung ist eine zu geringe Duktilität und speziell eine zu gering Röntgensichtbarkeit für den Einsatz als Stent-Werkstoff. Zudem gelten Cu und Al nicht als biokompatibel.

US 3,865,585 beschreibt eine nickelfreie Kobalt-Basislegierung mit Cr (26 - 31 Gew.%), Mo (4 - 6,5 Gew.%), Si (0 - 2 Gew.%), Fe (0 - 1 Gew.%), B (0 - 0,5 Gew.%), C (0 - 0,5 Gew.%), N (0.15 - 0.5 Gew.%), wobei der kumulierte Gehalt an C und N nicht höher als 0,7 Gew.% liegt.. Die Duktilität der Legierung ist mit weniger als 20% jedoch sehr gering und nicht für Stents geeignet.

US 3,837,838 beschreibt eine Kobalt-Basislegierung für die zahnmedizinische Anwendung mit Cr (20 - 32 Gew.%), Co (35 - 45 Gew.%), Ni und/oder Fe (30 - 40 Gew.%) sowie Ta oder Nb. Die Duktilität der Legierung liegt bei etwa 31% und ist somit nicht für Stents geeignet. Zwar ist die Dehngrenze von rund 300 MPa hinreichend gering, aber die Zugfestigkeit von 550 MPa ungenügend für die Zwecke eines Stents. Zudem liegt der Verfestigungskoeffizient (Dehngrenze/Zugfestigkeit) mit etwa 0.54 noch zu hoch.

DE 36 24 377 Al schlägt eine Kobalt-Basislegierung mit folgender Zusammensetzung für medizinische Implantate und festsitzende Dentalprothesen vor: Cr (15 - 24 Gew.%), Fe (2 - 15 Gew.%), Mo (3 - 10 Gew.%), N (0 - 0.05 Gew.%) und C (0 - 0.05 Gew.%). Die Duktilität ist mit rund 10% sehr gering und nicht für Stents geeignet.

"New DES Platforms" (D. Huibregtse et al, Cardiac Interventions Today, July/August 2011, 35-39) gibt einen Überblick über metallische Implantatwerkstoffe.

Demnach besteht anhaltender Bedarf an einem metallischen Implantatwerkstoff, der zur Herstellung von Stents geeignet ist.

Erfindungsgemäß wird eine Co-Basislegierung bereitgestellt, die auf Grund der folgenden Zusammensetzung den TWinning Induced Plasticity Effekt (TWIP-Effekt) als dominierenden Verformungsmechanismus aufweist:

| | |
|---|---|
| Cr: | 13.0 - 30.0 Gew.% |
| Mn: | 2.0 - 10.0 Gew.% |
| W: | 2.0 - 18.0 Gew.% |
| Fe: | 5.0 - 15.0 Gew.% |
| C: | 0.002 - 0.5 Gew.% |
| N: | 0 - 0.2 Gew.% |
| Si: | 0 - 2.0 Gew.% |
| Ni: | 0 - 5.0 Gew.% |

wobei sich die genannten Legierungskomponenten mit Kobalt als Rest und herstellungsbedingten Verunreinigungen auf 100 Gew.% summieren und für den Gehalt an Stickstoff und Kohlenstoff folgende Einschränkungen nach den Formeln (1) und (2), sowie für den Gehalt an Sauerstoff, Phosphor und Schwefel folgende Einschränkungen nach Formel (3) gelten

$$0,003 \leq C + N \leq 0,5 \qquad (1)$$

$$N/C \leq 1,00 \quad \text{für } 0.07 < C < 0.15 \quad (2)$$

$$O + P + S < 0,10 \qquad (3).$$

Die erfindungsgemäß verwendete Co-Basislegierung ist korrosionsbeständig, reibverschleißbeständig und besitzt eine hohe Festigkeit, die durch geeignete Wärmebehandlungen noch weiter erhöht werden kann, und gleichzeitig eine hohe Duktilität sowie ausgezeichnete Röntgensichtbarkeit. Die Legierungsbestandteile stabilisieren den Austenit, so dass die Legierung vorzugsweise vollständig in austenitischer (kubisch flächenzentriert; kfz) Modifikation vorliegt und erhöhen die Stapelfehlerenergie, wodurch der sogenannte TWIP-Effekt (TWinning Induced Plasticity), die Verformung auf Basis von Zwillingsbildung, befördert wird, die eine starke Verfestigung bei gleichzeitig hoher Duktilität ermöglicht.

Die erfindungsgemäß verwendeten Legierungen besitzen eine sehr hohe Zugfestigkeit von > 900 MPa, insbesondere > 1000 MPa, besonders bevorzugt > 1100 MPa. Die hohe Festigkeit ermöglicht es im Stent-Design dünne Strukturen zu realisieren, die dem Stent dennoch eine hohe Radialfestigkeit von > 1.5 bar (150 kPa) verleihen.

Die erfindungsgemäß verwendeten Legierungen besitzen ferner ein sehr ausgeprägtes Kaltverfestigungsverhalten ausgedrückt durch das Verhältnis $R_{p0,2}/R_m$ zwischen Dehngrenze $R_{p0,2}$ und Zugfestigkeit $R_m$, das besonders klein sein sollte. $R_{p0,2}/R_m$ sollte < 0.7, bevorzugt < 0.60, besonders bevorzugt < 0.5 sein. Diese Eigenschaft ist insbesondere bedeutsam für die Steuerung der Verformung bei der Stentaufweitung. Es wird ein homogenes Öffnungsverhalten angestrebt, das von den mechanischen Eigenschaften des Materials aber auch dem Stent-Design abhängt, welches wiederum mit mehr Freiheiten entwickelt werden kann, wenn die mechanischen Eigenschaften des Materials es zulassen.

Die erfindungsgemäßen Legierungen zeigen weiterhin eine ausgezeichnete Umformbarkeit bei Raumtemperatur. Der Verformungsgrad (Bruchdehnung $A_t$) liegt bei > 40% bevorzugt > 50%, insbesondere > 60%.

Im Ergebnis werden austenitische CoCrMnWFe-Legierungen bereitgestellt, die allenfalls einen geringen Nickelanteil aufweisen und als vordringlichen Verformungsmechanismus Zwillingsbildung zeigen (TWIP-Effekt). Aus diesem Material gefertigte Stents weisen eine höhere Radialfestigkeit, ein homogenes Öffnungsverhalten, größere Dilatationsreserven (Nominaldurchmesser + 0.5 mm) und eine bessere Crimpbarkeit (geringerer Durchmesser) bei gleichzeitig verringertem Stegquerschnitt (besseres Einheilverhalten) gegenüber herkömmlichen Stents aus. Auf Grund der Nickel-Freiheit oder des geringen Ni-Gehaltes resultiert eine stark verbesserte Biokompatibilität. Zudem ist die Beständigkeit gegen Lochfraß, gegen Abrieb und gegen Reibkorrosion insbesondere in den Situationen verbessert, in denen zwei oder mehrere Stents sich gegenseitig überlappen.

Die Ursache für den TWIP-Effekt liegt in den so genannten Stapelfehlern. Stapelfehler kann man sich als Verschiebung in regelmäßig aufeinandergestapelten Atomlagen vorstellen. An einem solchen Stapelfehler kann eine Kristallstruktur umklappen, so dass sich die Kristallebenen ab der Verschiebung genau in umgekehrter Reihenfolge stapeln. Beim Umklappen entsteht eine Spiegelebene, auf deren beiden Seiten die Kristallbereiche gespiegelt erscheinen. Man spricht von Zwillingsbildung. Um einen Zwilling zu bilden, muss die so genannte Stapelfehlerenergie (SFE) aufgebracht werden.

Um den gewünschten TWIP-Effekt zu erreichen sind zwei Aufgaben zu lösen:

a) Der austenitische Zustand ist bei Raumtemperatur zu stabilisieren und
b) die Stapelfehlerenergie der Legierungsmatrix sollte zwischen 15 und 50 mJ/m$^2$, bevorzugt zwischen 20 und 30 mJ/m$^2$ liegen [Rémy et al., Material Science and Engineering 26 (1976) 123-132].

Die erfindungsgemäße Legierung erreicht beides durch Legieren der Kobaltsbasis mit abgestimmten Gehalten entsprechend geeigneter Legierungselemente, die den Austenit stabilisieren eine hohe Löslichkeit besitzen und die Stapelfehlerenergie senken (z.B. W, Mo) bzw. abheben (z.B. C, N, Ni).

Elemente, die in Kobaltlegierungen verwendet werden, lassen sich in zwei Gruppen hinsichtlich ihrer Wirkung auf die Übergangstemperatur von kfz zu hexagonal dichtest gepackt (hex), also ihre Wirkung bezüglich der Stabilisierung des austenitischen Zustandes einteilen. So senken Al, B, C, Cu, Fe, Mn, Nb, Ni, Sn, Ti und Zr die Übergangstemperatur von kfz zu hex, sie stabilisieren den austenitischen Zustand, während Sb, As, Cr, Ge, Ir, Mo, Os, Pt, Re, Rh, Ru, Si, Ta und W die Übergangstemperatur anheben.

Die Elemente C, N, Mn und gegebenenfalls Ni dienen demnach der Stabilisierung des austenitischen Zustands.

Die erfindungsgemäße Legierung beinhaltet C und N in Summe bis maximal 0.5 Gew.% und minimal 0.003 Gew.%. Bevorzugt liegt die Summe im Bereich von 0.003 bis 0.3 Gew.%, insbesondere im Bereich von 0.003 bis 0.1 Gew.%.

Kohlenstoff besitzt eine den Austenit stabilisierende Wirkung und erhöht weiterhin die Stapelfehlerenergie äußerst effektiv. Kohlenstoff erhöht zudem die Festigkeit durch Bildung von Karbiden. Daher sollte der C-Gehalt nicht unter 0.002 Gew.% absinken. Andererseits ist darauf zu achten, dass es bei höheren C-Gehalten durch eine übermäßige Karbidbildung zu keiner Versprödung und oder Verringerung der Korrosionsbeständigkeit kommt. Daher sollte der C-Gehalt unter 0.5 Gew.% liegen. Der Anteil von C an der Legierung beträgt daher 0.002 bis 0.5 Gew.%, vorzugsweise 0.002 bis 0.15 Gew.%, besonders bevorzugt 0.002 bis 0.07 Gew.%..

In ähnlicher Weise bewirkt auch Stickstoff eine Stabilisierung des Austenit, eine Erhöhung der Stapelfehlerenergie und eine Steigerung der Festigkeit beziehungsweise auch der Härte durch Nitridbildung. Zudem kann Stickstoff die Karbidbildung bei höheren Kohlenstoffgehalten verhindern. Der Anteil von N an der Legierung kann in Abhängigkeit vom C-Gehalt bis zu 0.2 Gew.%, bevorzugt 0.15 Gew.%, besonders bevorzugt 0.05 Gew.% betragen. Insbesondere bei Kohlenstoffgehalten zwischen 0.07 und 0.15 Gew.% sollte Stickstoff in einem Verhältnis von 0.25 bis 1 zugesetzt werden.

Ni erhöht die Stapelfehlerenergie und fördert so die Ausbildung des gewünschten TWIP-Effektes. Ein Anteil von Ni an der Legierung darf aus Gründen der Biokompatibilität 5.0 Gew.% nicht übersteigen und liegt bevorzugt zwischen 2.0 bis 5.0 Gew.%, insbesondere zwischen 2.5 bis 3.5 Gew.%.

Eisen stabilisiert in Kobalt ab einem Gehalt von ca. 5 Gew.% [Onozuka et al., Journal of the Physical Society of Japan 37 (1974) 687-693] ebenfalls den Austenit. Für eine weitere Mischkristallhärtung kann Eisen ganz oder teilweise durch Mangan ersetzte werden. Ein Anteil von Fe an der Legierung beträgt 5.0 bis 15.0 Gew.%, insbesondere 9.5 bis 10.5 Gew.%.

Ein Anteil von Mn an der Legierung beträgt vorzugsweise 4.0 bis 10.0 Gew.%, insbesondere 4.5 bis 5.5 Gew.% beträgt.

Ferner ist bevorzugt, wenn ein Anteil von Cr an der Legierung bei 17.0 bis 25.0 Gew.%, insbesondere bei 19.0 bis 21.0 Gew.% liegt. In fester Lösung befindliches Chrom steigert die Festigkeit. Chrom ist aber auch im Wesentlichen für die Korrosions- und Oxidationsbeständigkeit verantwortlich. Ein hoher Chromgehalt bedeutet eine hohe Korrosionsbeständigkeit. Die erfindungsgemäßen Legierungen besitzen damit einen hohen Widerstand gegen lokale Korrosion, den so genannten Lochfraß.

Wolfram und Molybdän haben in etwa die gleiche Wirkung. Beide Elemente erhöhen die Festigkeit und verbessern den Korrosionsschutz, wirken jedoch auch der Stabilisierung des Austenit entgegen und senken zudem die Stapelfehlerenergie. Neben der Mischkristallhärtung und der Erhöhung der Reibbeständigkeit durch Wolfram, ist insbesondere im Anwendungsbereich als vaskuläre Gefäßstütze die erhöhte Werkstoffdichte durch Wolfram vorteilhaft, da dadurch eine gute Röntgensichtbarkeit erreicht wird. Daher ist es besonders bevorzugt, wenn ein Anteil von W an der Legierung 9.0 bis 18.0 Gew.%, insbesondere 14.5 bis 15.5 Gew.% beträgt.

Molybdän wirkt der Stabilisierung des Austenit entgegen und bildet zusammen mit Kobalt spröde intermetallische Phasen, die die Duktilität herabsetzten. Die Legierung ist daher frei von Molybdän.

Ein Anteil von Si an der Legierung ist vorzugsweise kleiner als 0.7 Gew.%, insbesondere liegt der Anteil bei 0.05 bis 0.5 Gew.%.

Weiterhin ist die Legierung weitgehend frei von Ti, Ta, Nb und Al.

Verunreinigungen, insbesondere O, S und P, können sowohl in Form von Oxiden, Sulfiden und Phosphiden ($Fe_3P$) als auch in fester Lösung die Duktilität mindern. Daher sollte der Gehalt jeweils unter 0,05 Gew.% liegen. In Summe sollte der Gehalt an O, S und P daher unter 0.10 Gew.%, bevorzugt unter 0.07 Gew.% liegen.

Die Erfindung betrifft ferner die Verwendung der vorgenannten Kobaltlegierung zur Herstellung eines Stents.

Die Legierungen sind analog den üblichen Herstellverfahren für Kobaltbasis-Legierungen herstellbar.

Ausführungsbeispiel 1

Co-20Cr-15W-10Fe-5Mn-3Ni-0.05C

Mechanische Eigenschaften im rekristallisierten Zustand nach einer Glühung bei ca. 1200°C mit anschließender Wasserabkühlung:

$$Rp0{,}2 = 500 - 550 \text{ MPa}$$

$$Rm = 1000 - 1050 \text{ MPa}$$

$$A > 60 \%$$

Ausführungsbeispiel 2

Co-22Cr-14W-8Fe-8Mn-0.15Si-0.15N-0.07C

Mechanische Eigenschaften im rekristallisierten Zustand nach einer Glühung bei ca. 1200°C mit anschließender Wasserabkühlung:

$$Rp0,2 = 520 - 570 \text{ MPa}$$

$$Rm = 1100 - 1150 \text{ MPa}$$

$$A > 50 \%$$

**Patentansprüche**

1. Kobaltlegierung der Zusammensetzung

| | |
|---|---|
| Cr: | 13.0 - 30.0 Gew.% |
| Mn: | 2.0 - 10.0 Gew.% |
| W: | 2.0 - 18.0 Gew.% |
| Fe: | 5.0 - 15.0 Gew.% |
| C: | 0.002 - 0.5 Gew.% |
| N: | 0 - 0.2 Gew.% |
| Si: | 0 - 2.0 Gew.% |
| Ni: | 0 - 5.0 Gew.% |

wobei sich die genannten Legierungskomponenten mit Kobalt als Rest und herstellungsbedingte Verunreinigungen auf 100 Gew.% summieren und für den Gehalt an Stickstoff und Kohlenstoff folgende Einschränkungen nach den Formeln (1) und (2), sowie für den Gehalt an Sauerstoff, Phosphor und Schwefel folgende Einschränkungen nach Formel (3) gelten

$$0,003 \leq C + N \leq 0,5 \qquad (1)$$

$$N/C \leq 1,00 \quad \text{für} \quad 0.07 < C < 0.15 \qquad (2)$$

$$O + P + S < 0,10 \qquad (3).$$

2. Legierung nach Anspruch 1, bei der ein Anteil von Cr an der Legierung 17.0 bis 25.0 Gew.% beträgt.

3. Legierung nach Anspruch 1, bei der ein Anteil von Mn an der Legierung 4.0 bis 10.0 Gew.% beträgt.

4. Legierung nach Anspruch 1, bei der ein Anteil von W an der Legierung 9.0 bis 18.0 Gew.% beträgt.

5. Legierung nach Anspruch 1, bei der ein Anteil von Fe an der Legierung 9.5 bis 10.5 Gew.% beträgt.

**6.** Legierung nach Anspruch 1, bei der ein Anteil von Si an der Legierung kleiner als 0.7 Gew.% ist.

**7.** Legierung nach Anspruch 1, bei der ein Anteil von C an der Legierung 0.02 bis 0.15 Gew.% beträgt.

**8.** Legierung nach Anspruch 1, bei der ein Anteil von C an der Legierung 0.07 bis 0.15 Gew.% beträgt und dabei N/C im Bereich von 0.25 bis 1 liegt.

**9.** Legierung nach Anspruch 1, bei der ein Anteil von Ni an der Legierung 2.0 bis 5.0 Gew.% beträgt.

**10.** Stent bestehend ganz oder in Teilen aus einer Kobaltlegierung der Zusammensetzung

| | |
|---|---|
| Cr: | 13.0 - 30.0 Gew.% |
| Mn: | 2.0 - 10.0 Gew.% |
| W: | 2.0 - 18.0 Gew.% |
| Fe: | 5.0 - 15.0 Gew.% |
| C: | 0.002 - 0.5 Gew.% |
| N: | 0 - 0.2 Gew.% |
| Si: | 0 - 2.0 Gew.% |
| Ni: | 0 - 5.0 Gew.% |

wobei sich die genannten Legierungskomponenten mit Kobalt als Rest und herstellungsbedingte Verunreinigungen auf 100 Gew.% summieren und für den Gehalt an Stickstoff und Kohlenstoff folgende Einschränkungen nach den Formeln (1) und (2), sowie für den Gehalt an Sauerstoff, Phosphor und Schwefel folgende Einschränkungen nach Formel (3) gelten

$$0{,}003 \le C + N \le 0{,}5 \qquad (1)$$

$$N/C \le 1{,}00 \quad \text{für } 0.07 < C < 0.15 \quad (2)$$

$$O + P + S < 0{,}10 \qquad (3).$$

**Claims**

**1.** A cobalt alloy having the composition
Cr: 13.0 - 30.0% by weight
Mn: 2.0 - 10.0% by weight
W: 2.0 - 18.0% by weight
Fe: 5.0 - 15.0% by weight
C: 0.002 - 0.5% by weight
N: 0 - 0.2% by weight
Si: 0 - 2.0% by weight
Ni: 0 - 5.0% by weight
wherein the aforementioned alloying components, with cobalt as the remainder, and manufacturing related impurities add up to 100% by weight, and wherein the following limitations according to formulas (1) and (2) apply for the content of nitrogen and carbon, and the following limitations according to formula (3) apply for the content of oxygen, phosphorous, and sulfur

$$0.003 \le C + N \le 0.5 \qquad (1)$$

$$N/C \le 1.00 \text{ for } 0.07 < C < 0.15 \qquad (2)$$

$$O + P + S < 0.10 \qquad (3).$$

2. The alloy according to claim 1, in which a portion of Cr in the alloy is 17.0 to 25.0% by weight.

3. The alloy according to claim 1, in which a portion of Mn in the alloy is 4.0 to 10.0% by weight.

4. The alloy according to claim 1, in which a portion of W in the alloy is 9.0 to 18.0% by weight.

5. The alloy according to claim 1, in which a portion of Fe in the alloy is 9.5 to 10.5% by weight.

6. The alloy according to claim 1, in which a portion of Si in the alloy is less than 0.7% by weight.

7. The alloy according to claim 1, in which a portion of C in the alloy is 0.02 to 0.15% by weight.

8. The alloy according to claim 1, in which a portion of C in the alloy is 0.07 to 0.15% by weight and N/C is in the range from 0.25 to 1.

9. The alloy according to claim 1, in which a portion ofNi in the alloy is 2.0 to 5.0% by weight.

10. A stent composed entirely or in parts of a cobalt alloy having the composition
    Cr: 13.0 - 30.0% by weight
    Mn: 2.0 - 10.0% by weight
    W: 2.0 - 18.0% by weight
    Fe: 5.0 - 15.0% by weight
    C: 0.002 - 0.5% by weight
    N: 0 - 0.2% by weight
    Si: 0 - 2.0% by weight
    Ni: 0 - 5.0% by weight
    wherein the aforementioned alloying components, with cobalt as the remainder, and manufacturing related impurities add up to 100% by weight, and wherein the following limitations according to formulas (1) and (2) apply for the content of nitrogen and carbon, and the following limitations according to formula (3) apply for the content of oxygen, phosphorous, and sulfur

$$0.003 \leq C + N \leq 0.5 \qquad (1)$$

$$N/C \leq 1.00 \text{ for } 0.07 < C < 0.15 \qquad (2)$$

$$O + P + S < 0.10 \qquad (3).$$

**Revendications**

1. Alliage de cobalt avec la composition suivante

| Cr: | 13,0 - 30,0 % en poids |
|---|---|
| Mn: | 2,0 - 10,0 % en poids |
| W: | 2,0 - 18,0 % en poids |
| Fe: | 5,0 - 15,0 % en poids |
| C: | 0,002 - 0,5 % en poids |
| N: | 0 - 0,2 % en poids |
| Si: | 0 - 2,0 % en poids |
| Ni: | 0 - 5,0 % en poids |

où lesdits composants de l'alliage, avec un reste consistant en du cobalt et des impuretés dues à la fabrication, donnent un total de 100 % en poids, et où les restrictions selon les formules (1) et (2) ci-dessous s'appliquent pour la teneur en azote et en carbone, et les restrictions selon la formule (3) ci-dessous s'appliquent pour la teneur en oxygène, en phosphore et en soufre :

$$0,003 \leq C + N \leq 0,5 \qquad (1)$$

$$N/C \leq 1,00 \text{ pour } 0,07 < C < 0,15 \qquad (2)$$

$$O + P + S < 0,10 \qquad (3).$$

2. Alliage selon la revendication 1, dans lequel une part de Cr représente de 17,0 à 25,0 % en poids dans l'alliage.

3. Alliage selon la revendication 1, dans lequel une part de Mn représente de 4,0 à 10,0 % en poids dans l'alliage.

4. Alliage selon la revendication 1, dans lequel une part de W représente de 9,0 à 18,0 % en poids dans l'alliage.

5. Alliage selon la revendication 1, dans lequel une part de Fe représente de 9,5 à 10,5 % en poids dans l'alliage.

6. Alliage selon la revendication 1, dans lequel une part de Si est inférieure à 0,7 % en poids dans l'alliage.

7. Alliage selon la revendication 1, dans lequel une part de C représente de 0,02 à 0,15 % en poids dans l'alliage.

8. Alliage selon la revendication 1, dans lequel une part de C représente de 0,07 à 0,15 % en poids dans l'alliage, et où N/C est compris entre 0,25 et 1.

9. Alliage selon la revendication 1, dans lequel une part de Ni représente de 2,0 à 5,0 % en poids dans l'alliage.

10. Stent entièrement ou partiellement constitué d'un alliage de cobalt avec la composition suivante

| | |
|---|---|
| Cr: | 13,0 - 30,0 % en poids |
| Mn: | 2,0 - 10,0 % en poids |
| W: | 2,0 - 18,0 % en poids |
| Fe: | 5,0 - 15,0 % en poids |
| C: | 0,002 - 0,5 % en poids |
| N: | 0 - 0,2 % en poids |
| Si: | 0 - 2,0 % en poids |
| Ni: | 0 - 5,0 % en poids |

où lesdits composants de l'alliage, avec un reste consistant en du cobalt et des impuretés dues à la fabrication, donnent un total de 100 % en poids, et où les restrictions selon les formules (1) et (2) ci-dessous s'appliquent pour la teneur en azote et en carbone, et les restrictions selon la formule (3) ci-dessous s'appliquent pour la teneur en oxygène, en phosphore et en soufre :

$$0,003 \leq C + N \leq 0,5 \qquad (1)$$

$$N/C \leq 1,00 \text{ pour } 0,07 < C < 0,15 \qquad (2)$$

$$O + P + S < 0,10 \qquad (3).$$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19704530 A1
- US 3865585 A
- US 3837838 A
- DE 3624377 A1

**In der Beschreibung aufgeführte Nicht-Patentliteratur**

- **D. HUIBREGTSE et al.** New DES Platforms. *Cardiac Interventions Today,* Juli 2011, 35-39
- **RÉMY et al.** *Material Science and Engineering,* 1976, vol. 26, 123-132
- **ONOZUKA et al.** *Journal of the Physical Society of Japan,* 1974, vol. 37, 687-693